# FASCICULE DE BREVET EUROPEEN

(11) **EP 1 236 394 B1**
(45) Date de publication et mention de la délivrance du brevet: **22.10.2003**
(21) Numéro de dépôt: 02290444.5
(22) Date de dépôt: 22.02.2002
(51) Int. Cl.: A01K 67/033

(54) **Procédé de culture d'acariens, préparation nutritive pour ce procédé, et préparation d'extraits allergéniques à partir de ces acariens**
Verfahren zur Züchtung von Hausstaubmilben, Herstellung der Nährkultur für dieses Verfahren und Verfahren zur Herstellung von allergenem Protein dieser Hausstaubmilben
Method for rearing house dust mites, preparation of the culture for such method and preparation of allergenic extracts from such mites

(30) Priorité: 01.03.2001 FR 0102835
(43) Date de publication de la demande: 04.09.2002
(73) Titulaire: Stallergenes S.A., 92183 Antony Cedex (FR)
(72) Inventeur: Andre, Claude, 69450 Saint Cyr Au Mont d'Or (FR); Batard, Thierry, 78000 Versailles (FR)
(74) Mandataire: Jacobson, Claude

(56) Documents cités:
- US-A- 6 129 935
- HALL C.C.: "COLLECTING AND REARING DERMATOPHAGOIDES FARINAE HUGHES, A MITE FROM HOUSEDUST" ANNALS OF ALLERGY, vol. 29, no. 2, février 1971 (1971-02), pages 81-85, XP001040913
- SCHOBER G.: "Synthetischer Hausstab als Substrat für allergenerzeugen Hausstaubmilben" ALLERGOLOGIE, vol. 14, no. 4, avril 1991 (1991-04), pages 140-143, XP001039819

## Description

La présente invention a trait à un procédé de culture d'acariens en vue de la production d'extraits allergéniques d'acariens, ainsi qu'à des formulations nutritives destinées à être mises en oeuvre dans ces procédés. Un procédé de culture d'acariens est décrit dans le brevet US 6129935.

Différentes espèces d'acariens sont utilisées pour préparer des extraits allergéniques mis en oeuvre dans des formulations d'allergologie pour servir, par exemple, de tests d'allergologie in vivo ou in vitro, ou encore de préparations désensibilisantes administrées aux patients.

Parmi ces acariens on trouve notamment les espèces suivantes : *Dermatophagoides pteronyssinus, Dermatophagoides farinae, Blomia kulagini* ou *tropicalis, Pyroglyphus africanus*, et *Euroglyphus maynei,* qui sont des acariens domestiques qui se nourrissent principalement de squames humaines.

On utilise classiquement, pour produire ces acariens, des milieux de culture contenant des squames humaines convenablement traitées et autoclavées en vue de l'inactivation des virus, bactéries et des agents transmissibles non conventionnels tels que les prions. Les acariens ainsi produits permettent d'obtenir, par extraction, des extraits allergéniques d'une grande qualité et pratiquement dépourvus d'allergènes d'autre origine susceptibles d'entraîner des réactions croisées pouvant mettre des tests en défaut ou susceptibles d'induire des allergies.

D'autres procédés de culture d'acariens sont connus, mettant en oeuvre des milieux nutritifs à base de protéines telles que des oeufs de crevettes ou de la poudre de foie de porc. Ces milieux permettent d'obtenir des productions satisfaisantes, mais comportent des substances non acariennes, notamment d'origine animale, pouvant être allergisantes et/ou à potentiel infectieux.

Bien que les procédés d'inactivation utilisés pour traiter les squames humaines destinées à la culture d'acariens soient d'une efficacité extrêmement élevée et que des contaminations d'agents infectieux conventionnels ou non soient hautement improbables, il est néanmoins souhaitable d'utiliser, pour cultiver les acariens, des milieux nutritifs d'origine non humaine et non animale et dépourvus d'éléments susceptibles d'être allergéniques.

La présente invention se propose donc de fournir un procédé de culture et de production d'acariens, et notamment des acariens des espèces précitées, limitant au maximum le risque de présence d'agents infectieux d'origine animale ou humaine.

Un autre objectif de l'invention est de fournir un tel procédé qui permette un rendement important en acariens.

Un autre objectif encore de l'invention est d'améliorer éventuellement l'antigénicité des acariens destinés à être utilisés ou extraits pour former les formulations finales.

Un autre objectif encore de l'invention est de permettre d'éliminer facilement une grande partie du milieu de culture.

L'invention a pour objet un procédé de culture et de production d'acariens, et notamment d'acariens appartenant à l'une au moins des espèces suivantes : *Dermatophagoides pteronyssinus, Dermatophagoides farinae, Blomia kulagini* ou *tropicalis, Pyroglyphus africanus*, et Euroglyphus maynei, caractérisée en ce que l'on cultive les -acariens sur milieu dépourvu d'éléments ou de protéines humaines ou animales et comprenant, en quantités efficaces, une pluralité d'acides aminés sous forme particulaire avec une granulométrie inférieure à 250 µm, ou sous forme lyophilisée.

La granulométrie recherchée d'acides aminés peut être obtenue par un broyage des acides aminés, individuellement ou en mélange.

De façon équivalente les acides aminés peuvent être obtenus par dissolution des acides aminés, puis-lyophilisation. Dans ce cas on préfère que la lyophilisation aboutisse à des particules de taille inférieure à 250 µm.

Bien entendu, dans le milieu selon l'invention, certains seulement des acides peuvent avoir été broyés et/ou lyophilisés, notamment en fonction de leurs caractéristiques physiques, par exemple leur solubilité, et, le cas échéant, certains acides peuvent être ajoutés tels quels dans le mélange.

L'invention repose sur la découverte que, si l'on utilise tels quels (sans broyage et/ou sans solubilisation-lyophilisation et/ou sans adjonction de sels) les acides aminés disponibles commercialement, les acariens se cultivent extrêmement mal et les rendements sont très faibles. De façon surprenante, les mélanges d'acides aminés ayant les caractéristiques définies dans l'invention aboutissent à des rendements comparables voire supérieurs aux rendements classiques utilisant des squames humaines.

Les mélanges d'acides aminés comportent, de préférence, la majorité ou la totalité des acides aminés constitutifs naturels des protéines. Par majorité on entend au moins 50%, par exemple 60 à 80%, des vingt acides aminés constitutifs naturels des protéines ou d'acides aminés équivalents assimilables. On peut cependant, également, ajouter des acides aminés non constitutifs, ou remplacer certains des acides aminés par des acides aminés non constitutifs de protéines.

Dans un mode de réalisation particulier, on peut avantageusement mettre en oeuvre un mélange d'acides aminés se rapprochant de la composition de la kératine ou de la couche cornée.

Cependant, dans des variantes de l'invention, on peut également utiliser des mélanges d'acides aminés se rapprochant de la composition des oeufs de crevettes ou du soja. Dans d'autres formulations on peut s'éloigner de cette distribution.

Les proportions respectives des acides aminés peuvent se rapprocher des proportions quantitatives des acides aminés dans des substances telles que la kératine, ou la couche cornée, les oeufs de crevettes ou le soja, mais une identité de distribution en proportion n'est nullement exigée, dès lors que les acides aminés individuels sont présents en quantité suffisante.

Le milieu nutritif qui comporte le mélange d'acides aminés du procédé selon l'invention peut également comporter d'autres éléments usuels de milieux nutritifs pour acariens, destinés soit à apporter un complément nutritif, soit à donner au milieu une texture propre au développement et à la multiplication des acariens, ainsi que des sels.

Ainsi on préfère incorporer, dans le milieu de culture, des germes de blé et/ou de la levure, notamment de la levure de boulangerie, et/ou de la cyanocobalamine et/ou de la d-biotine. Le milieu peut encore comporter d'autres vitamines.

Les germes de blé sont de préférence chauffés pour supprimer tout risque d'allergénicité.

Dans le procédé selon l'invention, le milieu de culture, contenant le mélange d'acides aminés, est amené à un degré d'humidité convenable, usuel pour les acariens que l'on cultive, et maintenu à la température habituelle convenable. Les durées de culture peuvent être, par exemple, de trois mois et l'on préfèrera des durées classiques de 2 à 5 mois.

L'invention a également pour objet les milieux de culture contenant les mélanges d'acides aminés selon l'invention.

L'invention a encore pour objet les procédés de préparations d'extraits ou de formulations d'allergènes obtenus à partir des acariens cultivés par le procédé selon l'invention.

D'autres avantages et caractéristiques de l'invention apparaîtront à la lecture de la description suivante, faite à titre d'exemple non limitatif.

Des cultures ont été conduites simultanément sur milieux acides aminés selon les exemples 1 et 3 et sur squames humaines selon l'exemple 2.

### Exemple 1 :

Cet exemple décrit un procédé de culture dans un milieu contenant une préparation commerciale d'acides aminés.

On prépare un milieu de culture contenant des germes de blé, de la cyanocobalamine, de la levure de boulangerie et de la D-biotine.

Les germes de blé sont autoclavés à 121°C pendant 20' puis broyés et tamisés sur un tamis de 250 µm.

La cyanocobalamine est broyée et tamisée sur un tamis de 250 µm de porosité.

La levure de boulangerie est chauffée à 122°C pendant 2 à 3', puis entre 100 et 135°C pendant 12" dans un tambour dont la vitesse de rotation est de 5 tours par minute, puis chauffée à 100°C pendant 15'.

La préparation commerciale d'acides aminés a été obtenue auprès de la société Frésénius-Kabi France S.A. Sa composition est la suivante, qsp 1 L d'eau PPI :
- L-alanine 3,8 g
- L-arginine 4,2 g
- acide L-aspartique 5,2 g
- L-cystéine chlorhydrate monohydratée exprimé en L-cystéine/L-cystine 1,7 g
- acide L-glutamique 11,5 g
- glycine 2,7 g
- L-histidine 3,1 g
- L-isoleucine 5,0 g
- L-leucine 6,7 g
- Chlorhydrate de L-Lysine, exprimé en L-lysine 5,0 g
- L-méthionine 2,4 g
- L-phénylalanine 7,0 g
- L-proline 10,3 g
- L-sérine 9,6 g
- L-thréonine 3,8 g
- L-tryptophane 1,3 g
- L-tyrosine 0,6 g
- L-valine 5,5 g
- Chlorure de calcium dihydraté 0,44 g
- Sulfate de magnésium heptahydraté 0,493 g
- Hydroxyde de sodium 2,6 g
- Hydroxide de potassium 0,70 g
- Chlorure de potassium 0,078 g

La solution est lyophilisée et le lyophilisat récolté est tamisé sur un tamis vibrant de 250 µm de porosité.

Le milieu proprement dit est préparé de la façon suivante :

Pour 600 g de milieu :

On pèse 252 g de germes de blé tamisé, 252 g de levures tamisées, 90 g de la solution d'acides aminés lyophilisée et tamisée, 5,4 g de cyanocobalamine tamisée et 0,6 g de D-biotine, l'ensemble étant homogénéisé dans un homogénéisateur puis tamisé sur un tamis de 400 µm de porosité. Le milieu est conditionné en flacons bouchés identifiés et peut être conservé en chambre froide à température comprise entre +2°C et +8°C.

La culture proprement dite est effectuée de la façon suivante :

Les milieux préparés sont ensemencés avec un échantillon de cultures d'ensemencement classique de *Dermatophagoides pteronisynus*. La culture s'effectue en flacons à une température de 25°C, à un degré humidité de 75%. Les prélèvements sont effectués à des époques différentes. Les milieux récoltés sont ensuite lyophilisés et extraits à 5% en solution de bicarbonate d'ammonium 4 g/1 pendant 24 heures à +4°C, puis centrifugés à 3000 tour par min. pendant 15 min. à +4°C. Le surnageant est récolté puis filtré sur filtre Millex HV de 0,45 µm de porosité (Millipore).

Dans les extraits obtenus on dose l'activité allergénique totale (par Rast-inhibition), les protéines (par la technique de Lowry et/ou la technique de Bradford), et les allergènes majeurs Der p 1 et Der p 2 (à l'aide des kits de dosage usuels).

Le résultat du Rast-inhibition est exprimé en IR/ml(IR : indice de réactivité), les extraits étant dosés en comparaison avec un extrait de référence dont l'activité est de 100 IR/ml.

### Exemple 2 :

Le milieu de l'exemple 2 est identique à celui de l'exemple 1 à la différence que la solution commerciale lyophilisée d'acides aminés est remplacée par une préparation de squames humaines.

Ces squames sont autoclavées dans des sachets à -134°C pendant 18'. Les squames sont ensuite déposées dans des plateaux en inox mis dans une étuve à 37°C pendant 1 à 2 semaines. Les squames sont ensuite broyées et tamisées avec des tamis de 500 et 250 µm.

La poudre de squames recueillie est ensuite traitée à l'acétone et laissée à décanter 24 heures. Le surnageant est éliminé. On procède ensuite à une dernière opération de lavage en acétone. On recueille le culot que l'on réparti en couches minces sur des plateaux et que l'on recouvre d'une feuille d'aluminium perforée. Cette substance est séchée sous hotte puis finalement tamisée avec un tamis vibrant de 250 µm.

Au lieu des 90 g de mélange d'acides aminés, le milieu de culture de cet exemple comprend 90 g de la préparation de squames humaines précitée.

### Exemple 3 :

Le milieu de culture comprend les germes de blé, la cyanocobalamine et la levure conformément aux exemples 1 et 2.

On prépare un mélange d'acides aminés de la façon suivante :

Dans un récipient on verse 10 litres d'eau distillée stérile et on solubilise les acides aminés suivants :
- L-alanine 17,2 g
- L-arginine 26,4 g
- L-cystéine chlorhydrate monohydratée 4,4 g
- glycine 49,5 g
- L-histidine 5,2 g
- L-isoleucine 13,2 g
- chlorhydrate de L-Lysine 20,0 g
- L-méthionine 8,0 g
- L-proline 8,8 g
- L-sérine 44,0 g
- L-thréonine 13,6 g
- L-valine 13,6 g
- L-tryptophane 1,3 g
- L-phénylalanine 20,8 g
- L-leucine 34,8 g
- acide L-glutamique 64,4 g
- acide L-aspartique 9,7 g

La solution est ensuite lyophilisée et le lyophilisât récolté est tamisé sur un tamis vibrant de 250 µm.

On broie séparément 6,5 g d'acide aspartique et 4,0 g de tyrosine.

Pour préparer le milieu on ajoute aux 252 g de germes de blé, 252 g de levures, 5,4 g de cyanocobalamine et 0,6 g de D-biotine, 79,5 g du mélange lyophilisé précité, 6,5 g d'acide aspartique broyé et 4,0 g de tyrosine broyée.

L'ensemble est homogénéisé puis tamisé sur 400 µm. Après ensemencement la culture est effectuée comme dans l'exemple 1 et 2.

Les résultats comparatifs des exemples 1 à 3 figurent dans le tableau 1 pour un premier cycle de culture, après 3 mois, et dans les tableaux 2 et 3 pour un second cycle de culture (les acariens cultivés sur un milieu donné sont ré-ensemencés sur le même milieu), après 2,5 mois et 3 mois, respectivement :

**TABLEAU 1**

| Activité allergénique totale, taux protéiques, de Der p 1 et de Der p 2 dans les extraits au 1/20^{ème} de *Dermatophagoides pteronyssinus* cultivé sur différents milieux après trois mois de culture. | | | |
|---|---|---|---|
| Milieu contenant | Acides aminés (exemple 1) | Squames humaines (exemple 2) | Acides aminés (exemple 3) |
| Activité allergénique totale (IR/ml) | 263 | 284 | 573 |
| Taux protéique(µg/ml ; technique de Bradford) | 537 | 544 | 217 |
| Taux protéique (µg/ml ; technique de Lowry) | 5689 | 6446 | 5538 |
| Der p 1 (µg/ml) | 187,5 | 231,5 | 69,0 |
| Der p 2 (µg/ml) | 2,0 | 2,5 | 10,0 |

**Tableau 2**

| Activité allergénique totale, taux protéique, de Der p 1 et de Der p 2 dans les extraits au 1/20ème de *Dermatophagoides pteronyssinus* cultivé pour un second cycle sur différents milieux après deux mois et demi de culture. | | | |
|---|---|---|---|
| Milieu contenant | Acides aminés (exemple 1) | Squames humaines (exemple 2) | Acides aminés (exemple 3) |
| Activité allergénique totale (IR/ml) | 617 | 713 | 195 |
| Taux protéique (µg/ml ; technique de Bradford) | 328 | 371 | 75 |
| Der p 1 (µg/ml) | 83,0 | 138,0 | 8,5 |
| Der p 2 (µg/ml) | 19,5 | 19,5 | 3,0 |

**Tableau 3**

| Activité allergénique totale, taux protéique, de Der p 1 et de Der p 2 dans les extraits au 1/20ème de Dermatophagoides pteronyssinus cultivé pour un second cycle sur différents milieux après trois mois de culture. | | | |
|---|---|---|---|
| Milieu contenant | Acides aminés (exemple 1) | Squames humaines (exemple 2) | Acides aminés (exemple 3) |
| Activité allergénique totale (IR/ml) | 631 | 486 | 192 |
| Taux protéique(µg/ml ; technique de Bradford) | 363 | 411 | 110 |
| Der p 1 (µg/ml) | 206,0 | 267,5 | 23,0 |
| Der p 2 (µg/ml) | 14,0 | 4,5 | 5,0 |

- On voit que si la culture sur acides aminés suivant l'exemple 3 donne de bons résultats lors d'un premier cycle de culture, elle donne de mauvais résultats sur un second cycle de culture. Cet exemple n'est donc pas adapté à la culture en routine des acariens.

En revanche, la culture sur acides aminés suivant l'exemple 1 semble bien adaptée, puisque non seulement les résultats sont satisfaisants après deux cycles de culture, mais encore ils sont très proches des résultats obtenus avec les squames humaines, qui constituent l'alimentation naturelle des acariens dont il est ici question. La relative faiblesse des résultats obtenus lors du premier essai est certainement imputable à un temps de culture trop important (une récolte à deux mois et demi aurait donné certainement de meilleurs résultats)..

## Revendications

1. Milieu de culture et de production d'acariens, et notamment d'acariens appartenant à l'une au moins des espèces suivantes : *Dermatophagoides pteronyssinus, Dermatophagoides farinae, Blomia kulagini* ou *tropicalis, Pyroglyphus africanus*, et *Euroglyphus maynei*, **caractérisé en ce qu'**il est dépourvu d'éléments ou de protéines humaines ou animales et qu'il comprend, en quantités efficaces, une pluralité d'acides aminés sous forme particulaire avec une granulométrie inférieure à 250 µm ou sous forme lyophilisée.

2. Milieu selon la revendication 1, **caractérisé en ce qu'**il comprend des acides aminés obtenus par un broyage d'acides aminés.

3. Milieu selon la revendication 2 **caractérisé en ce qu'**il comprend également des acides aminés lyophilisés et/ou tels quels du commerce.

4. Milieu selon la revendication 1 **caractérisé en ce qu'**il comprend des acides aminés lyophilisés et des acides aminés tels quels du commerce.

5. Milieu selon l'une des revendications 1 à 4 **caractérisé en ce qu'**il contient des sels.

6. Milieu selon l'une des revendications 1 à 5, **caractérisé en ce que** le mélange d'acides aminés comporte au moins 50% des acides aminés constitutifs naturels des protéines ou leurs équivalents.

7. Milieu selon l'une des revendications 1 à 6 **caractérisé en ce que** le mélange d'acides aminés reproduit le spectre des acides aminés constitutifs de la kératine ou de la couche cornée.

8. Milieu selon l'une des revendications 1 à 6, **caractérisé en ce que** le mélange d'acides aminés reproduit le spectre des acides aminés présents dans les oeufs de crevettes ou dans le soja.

9. Milieu selon l'une des revendications 7 et 8 **caractérisé en ce que** les proportions respectives des acides aminés sont proches des proportions quantitatives des acides aminés et des sels dans des substances telles que la kératine ou la couche cornée ou les oeufs de crevettes ou le soja.

10. Milieu selon l'une des revendications 1 à 9, **caractérisé en ce qu'**il comporte également d'autres éléments usuels de milieux nutritifs pour acariens, destinés à apporter un complément nutritif, et/ou à donner au milieu une texture propre au développement et à la multiplication des acariens.

11. Milieu selon la revendication 10, **caractérisé en ce qu'**il comporte en outre, des germes de blé et/ou de la levure, notamment de la levure de boulangerie, et/ou de la cyanocobalamine et/ou de la d-biotine.

12. Milieu selon l'une des revendications 1 à 11, **caractérisé en ce qu'**il peut contenir du soja.

13. Milieu selon l'une des revendications 1 à 12, **caractérisé en ce qu'**il comporte au moins 50% des acides aminés suivants :
- L-alanine
- L-arginine
- Acide L-aspartique
- L-cystéine/cystine
- Acide L-glutamique
- glycine
- L-histidine
- L-isoleucine
- L-leucine
- L-Lysine
- L-méthionine
- L-phénylalanine
- L-proline
- L-sérine
- L-thréonine
- L-tryptophane
- L-tyrosine
- L-valine

14. Milieu selon la revendication 13, **caractérisé en ce qu'**il comprend les acides aminés dans les proportions suivantes, pour un total de 93,711 g :
- L-alanine 3,8 g
- L-arginine 4,2 g
- acide L-aspartique 5,2 g
- L-cystéine chlorhydrate monohydratée exprimé en L-cystéine/L-cystine 1,7 g
- acide L-glutamique 11,5 g
- glycine 2,7 g
- L-histidine 3,1 g
- L-isoleucine 5,0 g
- L-leucine 6,7 g
- Chlorhydrate de L-lysine, exprimé en L-lysine 5,0 g
- L-méthionine 2,4 g
- L-phénylalanine 7,0 g
- L-proline 10,3 g
- L-sérine 9,6 g
- L-thréonine 3,8 g
- L-tryptophane 1,3 g
- L-tyrosine 0,6 g
- L-valine 5,5 g
- Chlorure de calcium dihydraté 0,44 g
- Sulfate de magnésium heptahydraté 0,493 g
- Hydroxyde de sodium 2,6 g
- Hydroxide de potassium 0,70 g
- Chlorure de potassium 0,078 g

15. Procédé de culture et de production d'acariens, et notamment d'acariens appartenant à l'une au moins des espèces suivantes : *Dermatophagoides pteronyssinus*, *Dermatophagoides farinae, Blomia kulagini* ou *tropicalis, Pyroglyphus africanus,* et *Euroglyphus maynei*, **caractérisé en ce que** l'on cultive les acariens sur un milieu selon l'une quelconque des revendications 1 à 14.

16. Procédé selon la revendication 15 **caractérisé en ce que** le milieu de culture, contenant le mélange d'acides aminés, est amené à un degré d'humidité-convenable, usuel pour les acariens que l'on cultive, et maintenu à la température habituelle convenable.

17. Procédé selon l'une des revendications 15 et 16 **caractérisé en ce que** l'on effectue la culture entre 2 et 5 mois.

18. Procédé d'obtention d'une préparation d'allergènes **caractérisé en ce que** l'on réalise une extraction des allergènes de la culture selon l'une des revendications 15 à 17.

## Patentansprüche

1. Medium zur Kultur und zur Produktion von Acari (Milben) und insbesondere von Acari mindestens einer der folgenden Spezies: *Dermatophagoides pteronyssinus, Dermatophagoides farinae, Blomia kulagini oder tropicalis, Pyroglyphus africanus und Euroglyphus maynei*, **dadurch gekennzeichnet, dass** es keine menschlichen oder tierischen Bestandteile oder Proteine enthält und dass es, in wirksamen Mengen, eine Mehrzahl von Aminosäuren in Teilchenform mit einer Granulometrie unter 250 µm oder in lyophilisierter Form umfasst.

2. Medium nach Anspruch 1, **dadurch gekennzeichnet, dass** es Aminosäuren umfasst, die durch eine Feinzerkleinerung von Aminosäuren erhalten werden.

3. Medium nach Anspruch 2, **dadurch gekennzeichnet, dass** es zudem lyophilisierte Aminosäuren und/oder Aminosäuren, wie handelsüblich, umfasst.

4. Medium nach Anspruch 1, **dadurch gekennzeichnet, dass** es lyophilisierte Aminosäuren und Aminosäuren, wie handelsüblich, umfasst.

5. Medium nach einem der Ansprüche 1 bis 4, **dadurch gekennzeichnet, dass** es Salze enthält.

6. Medium nach einem der Ansprüche 1 bis 5, **dadurch gekennzeichnet, dass** das Gemisch von Aminosäuren mindestens 50% der Aminosäuren, die natürliche Bestandteile von Proteinen sind, oder deren Äquivalente enthält.

7. Medium nach einem der Ansprüche 1 bis 6, **dadurch gekennzeichnet, dass** das Gemisch von Aminosäuren das Spektrum der Aminosäuren wiedergibt, die Bestandteile von Keratin oder der Lederhaut sind.

8. Medium nach einem der Ansprüche 1 bis 6, **dadurch gekennzeichnet, dass** das Gemisch von Aminosäuren das Spektrum der Aminosäuren wiedergibt, die in den Eiern von Garnelen oder in Soja vorliegen.

9. Medium nach einem der Ansprüche 7 und 8, **dadurch gekennzeichnet, dass** die jeweiligen Anteile der Aminosäuren nahezu die quantitativen Anteile der Aminosäuren und Salze in solchen Substanzen sind, wie Keratin oder der Lederhaut oder den Eiern von Garnelen oder Soja.

10. Medium nach einem der Ansprüche 1 bis 9, **dadurch gekennzeichnet, dass** es zudem andere, für Nährmedien für Acari übliche Bestandteile enthält, die eine Nahrungsergänzung beisteuern sollen und/oder dem Medium eine geeignete Beschaffenheit für die Entwicklung und Vermehrung von Acari geben sollen.

11. Medium nach Anspruch 10, **dadurch gekennzeichnet, dass** es außerdem Weizenkeime und/oder Hefe, insbesondere Bäckerhefe, und/oder Cyanocobalamin und/oder D-Biotin enthält.

12. Medium nach einem der Ansprüche 1 bis 11, **dadurch gekennzeichnet, dass** es Soja enthalten kann.

13. Medium nach einem der Ansprüche 1 bis 12, **dadurch gekennzeichnet, dass** es mindestens 50% der nachstehenden Aminosäuren enthält:
L-Alanin
L-Arginin
L-Asparaginsäure
L-Cystein/Cystin
L-Glutaminsäure
Glycin
L-Histidin
L-Isoleucin
L-Leucin
L-Lysin
L-Methionin
L-Phenylalanin
L-Prolin
L-Serin
L-Threonin
L-Tryptophan
L-Tyrosin
L-Valin

14. Medium nach Anspruch 13, **dadurch gekennzeichnet, dass** es die Aminosäuren in den folgenden Anteilen für insgesamt 93,711 g umfasst:
- L-Alanin 3,8 g
- L-Arginin 4,2 g
- L-Asparaginsäure 5,2 g
- L-Cysteinchlorhydratmonohydrat, ausgedrückt als L-Cystein/L-Cystin 1,7 g
- L-Glutaminsäure 11,5 g
- Glycin 2,7 g
- L-Histidin 3,1 g
- L-Isoleucin 5,0 g
- L-Leucine 6,7 g
- L-Lysinchlorhydrat, ausgedrückt als L-Lysin 5,0 g
- L-Methionin 2,4 g
- L-Phenylalanin 7,0 g
- L-Prolin 10,3 g
- L-Serin 9,6 g
- L-Threonin 3,8 g
- L-Tryptophan 1,3 g
- L-Tyrosin 0,6 g
- L-Valin 5,5 g
- Calciumchloriddihydrat 0,44 g
- Magnesiumsulfatheptahydrat 0,493 g
- Natriumhydroxid 2,6 g
- Kaliumhydroxid 0,70 g
- Kaliumchlorid 0,078 g

15. Verfahren zur Kultur und zur Produktion von Acari (Milben) und insbesondere von Acari mindestens einer der folgenden Spezies: *Dermatophagoides pteronyssinus, Dermatophagoides farinae, Blomia kulagini oder tropicalis, Pyroglyphus africanus und Euroglyphus maynei*, **dadurch gekennzeichnet, dass** die Acari in einem Medium nach einem der Ansprüche 1 bis 14 kultiviert werden.

16. Verfahren nach Anspruch 15, **dadurch gekennzeichnet, dass** das Kulturmedium, das das Gemisch von Aminosäuren enthält, auf einen angemessenen Feuchtigkeitsgrad gebracht wird, der für die kultivierten Acari üblich ist, und bei der angemessenen Normaltemperatur gehalten wird.

17. Verfahren nach einem der Ansprüche 15 und 16, **dadurch gekennzeichnet, dass** die Kultur für 2 bis 5 Monate durchgeführt wird.

18. Verfahren zur Gewinnung einer Zubereitung von Allergenen, **dadurch gekennzeichnet, dass** eine Extraktion von Allergenen aus einer Kultur nach einem der Ansprüche 15 bis 17 vorgenommen wird.

## Claims

1. Medium for culturing and producing dust mites, and in particular dust mites belonging to one at least of the following species: *Dermatophagoides pteronyssinus, Dermatophagoides farinae, Blomia kulagini* or *tropicalis, Pyroglyphus africanus* and *Euroglyphus maynei,* **characterised in that** it is devoid of human or animal elements or proteins and it comprises, in effective quantities, a plurality of amino acids in a particular form with a granulometry which is less than 250 µm or in a freeze-dried form.

2. Medium according to claim 1, **characterised in that** it comprises amino acids which are obtained by crushing amino acids.

3. Medium according to claim 2, **characterised in that** it comprises likewise freeze-dried amino acids and/or those commercially available.

4. Medium according to claim 1, **characterised in that** it comprises freeze-dried amino acids and amino acids as those commercially available.

5. Medium according to one of the claims 1 to 4, **characterised in that** it contains salts.

6. Medium according to one of the claims 1 to 5, **characterised in that** the mixture of amino acids comprises at least 50% of natural constituent amino acids of proteins or their equivalents.

7. Medium according to one of the claims 1 to 6, **characterised in that** the mixture of amino acids reproduces the spectrum of the constituent amino acids of keratin or of the dermis.

8. Medium according to one of the claims 1 to 6, **characterised in that** the mixture of amino acids reproduces the spectrum of amino acids present in shrimp eggs or in soya.

9. Medium according to one of the claims 7 and 8, **characterised in that** the respective proportions of amino acids are close to the quantitative proportions of amino acids and of salts in substances such as keratin or the dermis or shrimp eggs or soya.

10. Medium according to one of the claims 1 to 9, **characterised in that** it comprises likewise other usual elements of culture media for dust mites, which are intended to offer a culture complement, and/or to give the medium a texture which is suitable for the development and the multiplication of dust mites.

11. Medium according to claim 10, **characterised in that** comprises furthermore wheat germs and/or yeast, in particular baker's yeast, and/or cyanocobalamine and/or D-biotin.

12. Medium according to one of the claims 1 to 11, **characterised in that** it can contain soya.

13. Medium according to one of the claims 1 to 12, **characterised in that** it comprises at least 50% of the following amino acids:
- L-alanine
- L-arginine
- L-aspartic acid
- L-cysteine/cystine
- L-glutamic acid
- glycin
- L-histidine
- L-isoleucine
- L-leucine
- L-lysine
- L-methionine
- L-phenylalanine
- L-proline
- L-serine
- L-threonine
- L-tryptophan
- L-tyrosine
- L-valine

14. Medium according to claim 13, **characterised in that** it comprises the amino acids in the following proportions, for a total of 93.711 g:
- L-alanine 3.8 g
- L-arginine 4.2 g
- L-aspartic acid 5.2 g
- monohydrated L-cysteine hydrochloride expressed in L-cysteine/L-cystine 1.7 g
- L-glutamic acid 11.5 g
- glycin 2.7 g
- L-histidine 3.1 g
- L-isoleucine 5.0 g
- L-leucine 6.7 g
- L-lysine hydrochloride, expressed in L-lysine 5.0 g
- L-methionine 2.4 g
- L-phenylalanine 7.0 g
- L-proline 10.3 g
- L-serine 9.6 g
- L-threonine 3.8 g
- L-tryptophan 1.3 g
- L-tyrosine 0.6 g
- L-valine 5.5 g
- dihydrated calcium chloride 0.44 g
- heptahydrated magnesium sulphate 0.493 g
- sodium hydroxide 2.6 g
- potassium hydroxide 0.70 g
- potassium chloride 0.078 g

15. Method for culturing and producing dust mites, and in particular dust mites belonging to one at least of the following species: *Dermatophagoides pteronyssinus, Dermatophagoides farinae, Blomia kulagini* or *tropicalis, Pyroglyphus africanus* and *Euroglyphus maynei*, **characterised in that** the dust mites are cultured in a medium according to any of the claims 1 to 14.

16. Method according to claim 15, **characterised in that** the culture medium, containing the mixture of amino acids, is brought to a convenient degree of humidity which is usual for the dust mites being cultured, and maintained at the appropriate normal temperature.

17. Method according to one of the claims 15 and 16, **characterised in that** the culture is effected between 2 and 5 months.

18. Method for obtaining an allergenic preparation, **characterised in that** an extraction of allergens is produced from the culture according to one of the claims 15 to 17.
